# EUROPEAN PATENT APPLICATION

(11) **EP 2 123 248 A1**
(43) Date of publication of application: **25.11.2009**
(21) Application number: 09251350.6
(22) Date of filing: 21.05.2009
(51) Int. Cl.: A61K 8/24, A61K 8/49, A61K 8/65, A61K 8/73, A61K 8/81, A61K 8/98, A61L 27/24, A61L 27/54, A61Q 19/00, A61Q 19/08, A61K 8/36

(54) **A composition comprising a PPAR-gamma agonist and method of treating facial skin defect**

(30) Priority: 22.05.2008 US 125481
(71) Applicant: Johnson and Johnson Consumer Companies, Inc., Skillman, NJ 08558 (US)
(72) Inventor: Pappas, Apostolos Ph. D, Hillsborough, NJ 08844 (US); Seiberg, Miri, Princeton, NJ 08540 (US); Cavender, Druie E., Flemington, NJ 08822 (US)
(74) Representative: Kirsch, Susan Edith

(57) **Abstract**

This invention relates to a subcutaneous deliverable composition containing an agonist of the peroxisome proliferator-activated receptor-gamma, and a method for treating facial skin defects in a mammalian subject using the subcutaneous deliverable composition.

## Description

### Field of the Invention

This invention relates a composition and method of treating a facial skin defect by subcutaneous delivery of an agonist of the peroxisome proliferator-activated receptor-gamma (PPAR-γ).

### Background of the Invention

Peroxisome proliferator-activated receptor gamma (PPAR-γ) belongs to the nuclear hormone receptor subfamily of transcription factors. PPAR-γ is an essential regulator of adipocyte proliferation, differentiation, maintenance, and survival. See Journal of Biological Chemistry, 282(41), 29946-57, (2007).

Rosiglitazone is a member of a class of chemical compounds known as thiazolidinediones (TZD) and is a relatively selective agonist of PPAR-γ (N. Engl. J. Med. 351:1106-18, 2004). It has been hypothesized that rosiglitazone, among other TZD's, and non-TZD PPAR-γ agonists, might be useful for the treatment of the lipoatrophy that sometimes occurs in people receiving treatment for infection with a human immunodeficiency virus (HIV), or in people with diabetes. See Antivir. Ther., 8:199-207 (2003); Lancet, 363:429-38 (2003); AIDS, 17:770-2 (2003); Ann. Intern. Med., 143:337-46 (2005); J Clin Invest, 101:1354-61 (1998); and Diabetes, 46:1393-9 (1997). However, studies in such patients have yielded conflicting results. For example, systemic thiazolidinedione treatment has been shown to increase peripheral fat, but decrease visceral fat, of patients with insulin resistance and lipodystrophy. See "Efficacy and safety of troglitazone in the treatment of lipodystrophy syndromes", Ann. Intern. Med., 133:263-74 (2000).

A 24-week study showed that rosiglitazone via oral ingestion made no statistically significant difference in leg fat, limb fat, trunk fat, or total body fat between treatment groups and the placebo group in HIV subjects. See "A randomized, placebo-controlled trial of rosiglitazone for HIV lipoatrophy", J. Infect. Dis. 195: 1754-61 (2007).

WO 2007026356 discloses a method and compositions for treating or preventing a skin pathology or disorder associated with diabetes and/or aging by topical administration of at least one agent capable of restoring an impaired physiological condition of the skin associated with said skin pathology or disorder. WO 2007026356 suggests that such agents may be useful as anti-aging therapeutics by revitalizing the subcutaneous fat layer. However, there is evidence showing that preadipocytes from various body sites possess depot-specific characteristics. See "Aging in adipocytes: Potential impact of inherent, depot-specific mechanisms", Exptl. Gerontol. 42:463, 2007. For example, several PPAR-γ agonists induced more lipid droplets, and induced a greater increase in glycerol 3-phosphate dehydrogenase (G3PDH) enzymatic activity, in subcutaneous preadipocytes compared to omental preadipocytes. Importantly, this difference was observed despite similar levels of expression of the PPAR-γ protein; see "Activators of peroxisome proliferators-activated receptor γ have depot-specific effects on human preadipocyte differentiation", J. Clin. Invest. 100:3149, 1997). A very recent study has demonstrated that genome-wide expression profiles of primary preadipocytes from different body sites are distinct, and that these differences persist through multiple population doublings. See "Identification of depot-specific human fat cell progenitors through distinct expression profiles and developmental gene patterns", Am. J. Physiol. Endocrinol. Metab., 292:E298 (2007).

Furthermore, the fact that a drug is effective in oral or topical dosage form may not necessarily suggest that it may be efficacious and with tolerable side-effects when injected subcutaneously. For example, retinoids are used topically and orally for acne, but not as injectables due to intense tissue irritation and embryotoxicity. See G. Tzimas et al., "The area under the concentration-time curve of all-trans-retinoic acid is the most suitable pharmacokinetic correlate to the embryotoxicity of this retinoid in the rat", Toxicology And Applied Pharmacology 143, 436-444 (1997).

### Summary of the Invention

We have unexpectedly observed that agonists of the peroxisome proliferator-activated receptor-gamma, and in particular rosiglitazone potently and selectively enhances differentiation of facial preadipocytes *in vitro*, and, therefore, would enhance subcutaneous facial tissue differentiation *in vivo*.

Therefore, this invention relates to a composition and a method for treating facial skin defects in a mammalian subject by: a) providing a subcutaneous deliverable composition containing an agonist of the peroxisome proliferator-activated receptor-gamma, and a pharmaceutically acceptable carrier; b) identifying an area of facial skin in need of the treatment in said subject; c) delivering a safe and cosmetically effective amount of the composition subcutaneously to the identified area of facial skin. The subcutaneous delivery results in a reduction of the appearance of facial skin defects.

This invention also relates to a method of facial contouring in a mammalian subject by a) providing a subcutaneous deliverable composition containing an agonist of the peroxisome proliferator-activated receptor-gamma, and a pharmaceutically acceptable carrier; b) identifying an area of facial skin in need of the treatment; c) delivering a safe and cosmetically effective amount of the composition subcutaneously to the identified area of the skin. The subcutaneous delivery results in an improvement of facial contour around the identified area of facial skin.

This invention also relates to a subcutaneous deliverable composition for treating an appearance of skin defect in a mammalian subject. The subcutaneous deliverable composition contains: a) an agonist of the peroxisome proliferators-activated receptor-gamma, b) a dermal filling material; and c) a pharmaceutically acceptable carrier. The subcutaneous delivery of the composition results in a reduction of the appearance of skin defect around the identified area of facial skin

The subcutaneous deliverable composition of this invention may further contain a dermal filling material including, but not limited to, collagen, cross-linked collagen, hyaluronic acid, polylactic acid, calcium hydroxylapatite, cells, minced tissues, autologous transplanted cells or tissues, being intact or fragmented, gelatin, or the mixtures thereof.

Other features and advantages of the present invention will be apparent from the detailed description of the invention and from the claims.

### Detailed Description of Preferred Embodiments

Unless defined otherwise, all technical and scientific terms used herein have the same meaning as commonly understood by one of ordinary skill in the art to which the invention belongs. Also, all publications, patent applications, patents, and other references mentioned herein are incorporated by reference. Unless otherwise indicated, a percentage refers to a percentage by weight (i.e., %(W/W)).

As used herein, "subcutaneous delivery" means directly depositing in or underneath the skin, or in the subcutaneous fat layer, by use of an applicator such as a needle, a multi-needle array, an energy-based delivery system capable of subcutaneous delivery, a pressure-based delivery system capable of subcutaneous delivery, a needleless delivery system capable of subcutaneous delivery, or a similar medical device.

As used herein, "pharmaceutically-acceptable" means that the compound(s), carrier(s), or product(s), which the term describes are suitable for subcutaneous delivery without undue toxicity, incompatibility, instability, irritation, allergic response, and the like. This term is not intended to limit the ingredient or the product which it describes to use solely as a cosmetic (e.g., the ingredient or product may be used as a pharmaceutical agent).

As used herein, "cosmetically effective amount" means an amount of a physiologically active compound or composition sufficient for treating an appearance of facial skin defect or facial contouring in the condition to be treated, but low enough to avoid serious side effects. The cosmetically effective amount of the compound or composition will vary with the particular condition being treated, the age and physical condition of the end user, the severity of the condition being treated/prevented, the duration of the treatment, the nature of other treatments, the specific compound or product/composition employed, the particular pharmaceutically-acceptable carrier utilized, and like factors.

As used herein, "facial skin defect" includes, but is not limited, to scarred skin, wrinkled skin, furrowed skin, folding skin, sagging skin, skin atrophy from disease or trauma, defects secondary to skin grafting or other surgically-induced irregularities, skin defects resulting from an accident or trauma to the skin, and irregularity of skin.

As used herein, "treating an appearance of facial skin defect" means preventing, reducing, improving or eliminating the appearance of facial skin defects including, but not limited to, scarred skin, wrinkled skin, furrowed skin, folding skin, sagging skin, skin atrophy from disease or trauma, skin defects resulting from an accident or trauma to the skin, defects secondary to skin grafting or other surgically-induced irregularities, and irregularity of skin.

As used herein, "facial contouring" means adjusting, shaping, reforming or changing the facial features to a more youthful, full, and healthy look and to ameliorate the appearance of skin defects. Facial features include, but are not limited to, frown or glabellar line, acne scars, cheek depressions, vertical or perioral lip lines, marionette lines or oral commissures, worry or forehead lines, crow's feet or periorbital lines, nasolabial folds, smile lines, facial scars, lips and the like.

It is believed that one skilled in the art can, based upon the description herein, utilize the present invention to its fullest extent. The following specific embodiments are to be construed as merely illustrative, and not limitative of the remainder of the disclosure in any way whatsoever.

### Subcutaneous Compositions

The subcutaneous compositions useful in this invention contain formulations suitable for subcutaneous application. In one embodiment, the composition contains an agonist of PPAR-γ, and a pharmaceutically acceptable carrier.

The peroxisome proliferator-activated receptor-gamma as used herein, abbreviated as PPAR-γ, and also known as NR1C3 (nuclear receptor subfamily 1, group C, member 3), means the gamma (γ) subtype of the nuclear receptor. PPAR proteins form heterodimers with the retinoid X receptors, and these heterodimers regulate transcription of various genes. The protein encoded by the PPAR-gamma gene is known to be a positive regulator of adipocyte differentiation.

### Agonist of the peroxisome proliferator-activated receptor-gamma

As used herein, "agonist of the peroxisome proliferator-activated receptor-gamma " means a molecule, or a mixture of agents containing such a molecule (e.g. a botanical extract), that directly interacts with the PPAR-γ protein, and stimulates its interaction with retinoid X receptors and/or its target genes, to produce a physiological effect.

Agonists of PPAR-γ include, but are not limited to, rosiglitazone, ciglitazone troglitazone, englitazone, pioglitazone, linoleic acid metabolites of linoleic and arachidonic acid, and the mixtures thereof.

In one embodiment, the agonist of the peroxisome proliferator-activated receptor-gamma constitutes from about 0.00001% to about 5%, by weight, of the composition, more preferably from about 0.001% to about 0.1% by weight of the composition, and most preferably from about 0.01% to about 0.1% by weight of the composition.

In another embodiment, the agonist of the peroxisome proliferator-activated receptor-gamma include botanical and natural extracts which are known to enhance adipocyte differentiation. Such extracts, or fractions thereof, might be known activators of the PPARγ pathway (e.g. Pulpactyl, an extract from Artemisia abrotanum; Southernwood)), or might be known for their ability to enhance lipid production in experimental systems or in humans (e.g. Einkorn, an extract from Triticum monococcum).

### Pharmaceutically acceptable carriers

One or more pharmaceutically acceptable carriers may be present in the formulations of the present invention. Pharmaceutically-acceptable agents for subcutaneous delivery are well-known; examples of descriptions of such agents include: 1) Handbook on Injectable Drugs, 14th Edition (published by the American Society of Health-System Pharmacists (ASHP); and 2)the "inactive ingredients for approved drug products" database published online by the Center for Drug Evaluation and Research (CDER) at the U.S. Food and Drug Administration (FDA)

Suitable carriers of this invention include, but are not limited to, water, ethanol, isopropanol, 1,2-propanediol, glycerin, benzyl alcohol, dimethylisosorbide, triacetin, glycol ethers, propylene glycol and polyethylene glycol (PEG). Particularly preferred solvents include PEG having an average molecular weight between about 200 and about 400, castor oil, triacetin, dimethylisosorbide, ethanol, and water, and combinations thereof. The pharmaceutically-acceptable carrier constitutes from about 50% to about 99.99%, by weight, of the composition, more preferably from about 80% to about 95%, by weight, of the composition.

Various compounds may be added to the formulation to alter osmolarity and/or pH to acceptable levels. These include, but are not limited to, mannitol, sucrose, calcium chloride, sodium chloride, sodium phosphate monobasic, sodium phosphate dibasic, sodium hydroxide, and hydrochloric acid.

A surfactant may be added to the composition. Exemplary surfactants include nonionic surfactants such as polysorbates (e.g. polysorbates 20, 80, such as Tween®20, Tween®80) or poloxamers (e.g. poloxamer 188). The amount of surfactant added is such that it reduces aggregation of the formulation and/or minimizes the formation of particulates in the formulation, without reducing the biological activity. The surfactant may be present in the formulation in an amount from about 0.001% to about 0.5%, preferably from about 0.005% to about 0.1% ,and most preferably from about 0.01% to about 0.05%.

### Dermal filling material

The composition of this invention may further contain a dermal filling material.

As used herein, "dermal filling material" means a material that is not immediately resorbed and degraded in tissues, and which is used as a deliverable for cosmetic and aesthetic needs. The so-called "injectable fillers" are used by cosmetic, dermatological and plastic surgeons to reduce the depth of skin folds, to reduce wrinkles, to fill tissue defects, to reduce the visibility of scars, to reduce or correct atrophy from disease or trauma, to correct defects secondary to skin grafting or other surgically-induced irregularities, and to improve or eliminate other soft tissue defects or deficiencies.

The dermal filling material of this invention includes, but is not limited to, collagen, cross-linked collagen, hyaluronic acid, poly lactic acid, Calcium hydroxyl apatite, polymers, cells, minced tissues, autologous transplanted cells or tissues, being intact or fragmented, gelatin, or the mixtures thereof. Rosiglitazone binds tightly to serum proteins (99.8% bound in vivo) and might also bind to other proteins or protein-containing fillers (e.g., collagen, gelatin, cells, minced tissue, autologous transplanted cells or tissues, being intact or fragmented). Such binding could enhance the efficacy of rosiglitazone (and perhaps other PPAR-γ agonists) for the purpose of this invention by slowing the diffusion of the agonist away from the site of delivery.

In one embodiment, the dermal filling material constitutes from about 1% to about 10%, of the composition, more preferably from about 1.5% to about 8%, by weight, of the composition, and most preferably from about 2.5% to about 4.5% by weight of the composition.

### Cross-linked collagen

As used herein, "cross-linked collagen" means a polymer composite of collagen molecules that are connected together. Cross-links are covalent bonds linking one polymer chain to another, which are formed by chemical reactions that are initiated by heat and/or pressure, or by the mixing of an unpolymerized or partially polymerized unit with specific chemicals called crosslinking reagents. Crosslinking inhibits the close packing of polymer chains and prevents the formation of crystalline regions. A cross-linked biological structure such as cross-linked collagen has restricted molecular mobility which limits the extension of the polymer material, and is less prone to degradation than the collagen monomer.

Suitable cross-linked collagen of this invention include, but is not limited to, collagen molecules of natural or synthetic sources that are cross-linked by e.g., heat, solvents, organic agents, coagulation agents, sugars, glycosaminoglycans, glutaraldehydes and the like.

### Sugar cross-linked collagen

As used herein, "sugar cross-linked collagen" means collagen molecules that are chemically connected by reacting with sugars. One non-limiting example of sugar cross-linked collagen is a collagen cross-linked by the Glymatrix™ technology, which is based on a non-enzymatic glycation process. This cross-linking technology utilizes D-ribose as a cross linking agent.

In one embodiment, the sugar cross-linked collagen constitutes from about 1% to about 10%, of the composition, more preferably from about 1.5% to about 8%, by weight, of the composition, and most preferably from about 2.5% to about 4.5% by weight of the composition.

### Agents capable of modulating the activity of an insulin-signaling pathway

The composition of this invention may further contain one or more agents capable of modulating the activity of an insulin-signaling pathway.

As used herein, "agents capable of modulating the activity of an insulin signaling pathway" means agents that could affect physiological processes regulated by insulin. Insulin is involved in the regulation of intracellular and blood glucose levels and the avoidance of diabetes. Insulin binds to its receptor leading to the phosphorylation of insulin receptor substrates. These, in turn, phosphorylate other intermediates and activate the Ras and the AKT signaling pathways, leading to changes in gene transcription, resulting in modulation of glucose levels. The agents capable of modulating the activity of the insulin signaling pathway of this invention includes, but is not limited to, insulin, dexamethasone, 3-Isobutyl-1-methylxanthine (IBMX), corticosteroids, and non-specific phosphodiesterase inhibitors.

### Formulations

The compositions useful in the present invention involve formulations suitable for administering to the target tissues. The compositions of this invention may be made into a wide variety of product types that include but are not limited to solutions, gels, emulsions, suspension, microemulsions, nanoemulsions, liquid drops, liposomes, slow-releasing materials, polymers or monomers and polymerizing agents, and the like.

The compositions useful in the present invention can be formulated as solutions. Solutions should preferably include an aqueous solvent. Such compositions may further contain about 1% to about 30% organic solvent, although this may vary dependent upon the formulation. Non-limiting examples of such solvents include ethanol, propylene glycol, polyethylene glycol, and mixtures thereof.

The subcutaneous compositions useful in the present invention may also be formulated as emulsions. If the carrier is an emulsion, from about 1% to about 10% (preferably from about 2% to about 5%) of the carrier should be made up of one or more emulsifiers. Emulsifiers may be nonionic, anionic or cationic. Suitable emulsifiers may be found in, for example, the 2008 International Cosmetic Ingredient Dictionary and Handbook, 12th Edition published by the Personal Care Products Council).

Single emulsion preparations, such as the oil-in-water type and water-in-oil type are well-known in the cosmetic art and are useful in the subject invention. Multiphase emulsion compositions, such as the water-in-oil-in-water type are also useful in the subject invention. In general, such single or multiphase emulsions contain water, emollients, and emulsifiers as essential ingredients.

The compositions of this invention may be formulated as a gel (e.g., an aqueous gel using a suitable gelling agent(s)). Suitable gelling agents for aqueous gels include, but are not limited to, natural gums, acrylic acid and acrylate polymers and copolymers, and cellulose derivatives (e.g., hydroxymethyl cellulose and hydroxypropyl cellulose). Suitable gelling agents for oils (such as mineral oil) include, but are not limited to, hydrogenated butylene/ethylene/styrene copolymer and hydrogenated ethylene/propylene/styrene copolymer. Such gels typically comprises between about 0.1% and 5%, by weight, of such gelling agents.

The compositions useful in this invention may contain, in addition to the aforementioned components, a wide variety of additional oil-soluble, organic solvent-soluble, and/or water-soluble materials conventionally used in compositions for use on skin at their art-established levels.

### Additional Cosmetically Active Agents

In one embodiment, the compositions according to this invention may further contain one or more additional cosmetically active agent(s) as well as the above-mentioned components. What is meant by a "cosmetically active agent" is a compound, which may be a synthetic compound or a compound extracted, isolated, purified or concentrated from a natural source, or a natural extract containing a mixture of compounds, that has a cosmetic or therapeutic effect on the tissue, including, but not limited to: antimicrobial agents such as anti-yeast, anti-fungal, and anti-bacterial agents, anti-inflammatory agents, anti-aging agents, anti-parasite agents, antioxidants, keratolytic agents, nutrients, vitamins, minerals, energy enhancers, pH-changing agents and the like.

Examples of vitamins that may be constituents of the compositions of this invention include, but are not limited to, vitamin A, vitamin Bs such as vitamin B3, vitamin B5, vitamin B7 and vitamin B12, vitamin C, vitamin K, vitamin E such as alpha, gamma or delta-tocopherol, and their derivatives (such as salts and esters) and mixtures thereof.

Examples of antioxidants which may be utilized in the compositions and methods of this invention include, but are not limited to, water-soluble antioxidants such as sulfhydryl compounds and their derivatives (e.g., sodium metabisulfite and N-acetyl-cysteine), lipoic acid and dihydrolipoic acid, resveratrol, lactoferrin, and ascorbic acid and ascorbic acid derivatives (e.g., ascorbyl palmitate and ascorbyl polypeptide). Oil-soluble antioxidants suitable for use in the compositions of this invention include, but are not limited to, butylated hydroxytoluene, retinoids (e.g., retinol and retinyl palmitate), different types of tocopherols (e.g., alpha-, gamma-, and delta-tocopherols and their esters such as acetate) and their mixtures, tocotrienols, and ubiquinone. Natural extracts containing antioxidants suitable for use in the compositions of this invention include, but are not limited to, extracts containing flavinoid, isoflavinoid, and their derivatives such as genistein and diadzein (e.g., such as soy and clover extracts, extracts containing resveratrol and the like.

### Delivery of agents

In one embodiment, the composition of this invention is delivered by subcutaneous injection. A subcutaneous injection is a method of delivering agents into, for example, the fat layer between the skin and the muscle, using a syringe filed with the agent, which is attached to a needle. In one embodiment of this invention, a syringe and needle injection device may be used to deliver a suspension that contains a PPAR gamma agonist, with or without a dermal filler, into the individual's fat layer of the skin.

Needleless injection devices are disclosed in US patents 7,320,677, 5,938,637,and 6,447,475, which are incorporated herein by reference. Such needleless injection devices are particularly useful to deliver material to skin and muscles. In one embodiment of this invention, a needleless injection device may be used to propel, for example, a suspension that contains a PPAR gamma agonist toward the surface of the individual's skin. The material is propelled at a sufficient velocity such that upon impact with the skin it penetrates the surface of the skin, and permeates the skin tissue.

Iontophoretic drug delivery systems are disclosed, e.g., under the trademark of IONSYS(TM), and in US patent 4,281,709, which is incorporated herein by reference. Such delivery systems include a patch with a medicated surface or reservoir, and a controller, which supplies an electric current, resulting in an iontophoretic drug delivery. In one embodiment of this invention, an iontophoretic device may be used to deliver e.g. a solution or a suspension that contains a PPAR gamma agonist into the individual's skin.

Methods of enhanced delivery of active agents into the skin (e.g., for treating cellulite) using conductive power are described in US20040267232, US20050148996 and US20070060862, which are incorporated herein by reference. Such devices have a barrier, membrane-contacting surface, a power source including conductive electrodes and a reservoir. In one embodiment of this invention, such a delivery device may be used to deliver e.g. a solution or a suspension that contains a PPAR gamma agonist into the individual's skin.

### Other Materials

Various other materials may also be present in the compositions useful in the subject invention. These include proteins and polypeptides, preservatives and an alkaline agent. Examples of such agents are disclosed in the 2008 International Cosmetic Ingredient Dictionary and Handbook, 12th Edition published by the Personal Care Products Council).

The present invention is further defined in the following Examples. It should be understood that these Examples, while indicating preferred embodiments of the invention, are given by way of illustration only. From the above discussion and these Examples, one skilled in the art can ascertain the essential characteristics of this invention, and without departing from the spirit and scope thereof, can make various changes and modifications of the invention to adapt it to various uses and conditions.

### EXAMPLES

### Example 1. Subcutaneous Compositions

The following are examples of the compositions of this invention. As used in the subsequent Examples, the weight percentage of composition refers to the weight of the liquid extract.

**Table 1. Rosiglitazone formulations**

| a. Rosiglitazone is dissolved in 50:50 ethanol/saline. | |
|---|---|
| **Ingredients** | **Percentage % (w/v)** |
| Rosiglitazone | 0.00001 |
| ethanol | 50 |
| saline | 49.99999 |

| b. Rosiglitazone and vitamins B5 and B7 dissolved in 20:30: 50 Dimethylsulfoxide/ethanol /DI water with saline. | |
|---|---|
| **Ingredients** | **Percentage % (w/v)** |
| Rosiglitazone | 0.01 |
| Dimethylsulfoxide | 20 |
| Ethanol | 30 |
| saline | 48.99 |
| Vitamin B7 | 0.5 |
| Vitamin B5 | 0.5 |

| c. Rosiglitazone and vitamin E are dissolved in 50:50 castor oil/saline | |
|---|---|
| **Ingredients** | **Percentage % (w/v)** |
| Rosiglitazone | 5.0 |
| castor oil | 46.75 |
| saline | 48.15 |
| Vitamin E | 0.1 |

| d. Rosiglitazone and Linoleic Acid are dissolved in 20:30:50 Castor oil/ethanol /phoapahte-buffered saline (PBS). | |
|---|---|
| **Ingredients** | **Percentage % (w/v)** |
| Rosiglitazone | 0.01 |
| Castor oil | 20 |
| Ethanol | 30 |
| PBS^{*} | 48.99 |
| linoleic acid | 1 |

| e. Rosiglitazone is mixed in PBS to form a suspension. | |
|---|---|
| **Ingredients** | **Percentage % (w/v)** |
| Rosiglitazone | 0.005 |
| PBS | 99.995 |

| f. Rosiglitazone is mixed with sodium(Na)alginate to form a slow-release formulation. | |
|---|---|
| **Ingredients** | **Percentage % (w/v)** |
| Rosiglitazone | 0.001 |
| ethanol | 47 |
| saline | 47.999 |
| Water soluble Na-alginate | 5 |

| g. Rosiglitazone is mixed with a biodegradable polymer (poly-lactide/glycolide) to form slow-release microparticles; the microparticles are then suspended in saline. | |
|---|---|
| **Ingredients** | **Percentage % (w/v)** |
| Rosiglitazone | 0.01 |
| poly(glycolide/lactide) | 1 |
| saline | 98.99 |

**Table 2. Rosiglitizone and sugar cross-linked collagen formulations**

| a. Rosiglitazone, Evolence and vitamin E are dissolved in 50:50 ethanol/DI water with phosphate-buffered saline (PBS). (Evolence is a sugar cross-linked collagen) | |
|---|---|
| **Ingredients** | **Percentage % (w/v)** |
| Rosiglitazone | 0.5 |
| Evolence* | 10 |
| ethanol | 44 |
| PBS | 45.4 |
| Vitamin E | 0.1 |
| • Evolence is collagen product cross-linked via the Glymatrix technology | |

| b. Rosiglitazone, Evolence and vitamins B5 and B7 are dissolved in 20:30:44 Dimethylsulfoxide/ethanol /DI water with saline. | |
|---|---|
| **Ingredients** | **Percentage % (w/v)** |
| Rosiglitazone | 5 |
| Evolence | 1 |
| Dimethylsulfoxide | 20 |
| Ethanol | 30 |
| saline | 42.5 |
| Vitamin B5 | 0.75 |
| Vitamin B7 | 0.75 |

| c. Rosiglitazone, Evolence and vitamin E are dissolved in 50:50 castor oil/DI water with saline | |
|---|---|
| **Ingredients** | **Percentage** % **(w/v)** |
| Rosiglitazone | 1.0 |
| Evolence | 2 |
| castor oil | 47.75 |
| saline | 49.15 |
| Vitamin E | 0.1 |

| d. Rosiglitazone is mixed with Evolence in PBS to form a suspension. | |
|---|---|
| **Ingredients** | **Percentage % (w/v)** |
| Rosiglitazone | 0.05 |
| Evolence | 5 |
| PBS | 94.95 |

| e. Rosiglitazone is mixed with Evolence, Linoleic Acid and Vitamin E in PBS to form a suspension. | |
|---|---|
| **Ingredients** | **Percentage % (w/v)** |
| Rosiglitazone | 0.1 |
| Evolence | 5 |
| PBS | 94.7 |
| Linoleic Acid | 0.1 |
| vitamin E | 0.1 |

### Example 2. Rosiglitazone potently and selectively enhances differentiation of facial preadipocytes

Human primary preadipocytes from facial and abdominal skin samples, obtained with informed consent, were isolated and cultured following a published procedure (Crandall et al, Endocrinology 140:154-8, 1999). Briefly, samples from abdominal or facial operations were subjected to enzymatic digestion in Krebs-Ringer-bicarbonate buffer (pH 7.4) containing 6 mM glucose and 2 mg/mL collagenase. After this initial enzymatic digestion, the content of the flask was passed through a sterile, 230-micron stainless steel tissue sieve (Cellector, Bellco Glass Inc., Vineland, NJ) into a 50-mL sterile, plastic test tube. Undigested stromal-vascular tissue trapped on the sieve was discarded, while the infranatant containing the preadipocyte fraction was collected, passed into another sterile tube, and the collagenase neutralized with an equal volume of growth medium containing Medium 199, 10% heat-inactivated FCS, and 1% antibiotic-antimycotic (Life Technologies, Grand Island, NY). After centrifugation, the pellet was resuspended in growth medium, filtered, transferred to a sterile tissue culture flask, and maintained in an incubator at 37 C, 5% CO₂. Cell attachment was allowed for 16-20 h, after which floating cells were removed by aspiration, followed by addition of fresh growth medium.

All differentiation agents (IBMX, dexamethasone, insulin, and rosiglitazone) were added to the culture media 24 hours after the cells were seeded. The media were changed three days later and supplemented with fresh preparations of the respective agents. After the seventh day in culture, IBMX and rosiglitazone were removed from the culture media, and the cells were kept for 5 to 10 more days for observation of lipid droplet formation, which is indicative of the cells' ability to differentiate. Between the twelfth and the fifteenth day, images of cultured cells and their produced lipid droplets were acquired by an inverted microscope. Subsequently, the cultures were incubated with oil red O (a dye that binds to neutral lipids); the dye was then extracted and quantified by spectrophotometry. Increased oil red O staining correlated with more lipid production, and therefore with higher level of differentiation. The results given in the table below are a summary from three separate experiments:

**Table 3**

| Source of cells | *Treatment | Degree of differentiation |
|---|---|---|
| **facial** | **-** | **-** |
| | **I+D+X** | **++** |
| | **I+D+X+R** | **++++** |
| **abdominal** | **-** | **-** |
| | **I+D+X** | **+** |
| | **I+D+X+R** | **+++** |

| | | |
|---|---|---|
| • I - insulin; D = dexamethasone; X = IBMX; R = rosiglitazone - no differentiation + minimal differentiation ++ low differentiation +++ moderate differentiation ++++ high differentiation | | |

Table 3 demonstrates that facial preadipocytes are more responsive to rosiglitazone-containing differentiation compositions, relative to abdominal preadipocytes. This example suggests that facial preadipocytes treated with such compositions would differentiate more completely than would non-facial preadipocytes.

Importantly, as shown in Table 4 below, the combination of rosiglitazone and insulin only, induced a similar degree of differentiation as all four agents together; therefore, rosiglitazone can replace IBMX and dexamethasone in the induction of pre-adipocyte differentiation at least for facial preadipocytes.

**Table 4**

| Source of cells | Treatment^{*} | Degree of differentiation |
|---|---|---|
| **facial** | **-** | **-** |
| | **I+D+X** | **++** |
| | **I+R** | **++** |

| | | |
|---|---|---|
| I - insulin; D = dexamethasone; X = IBMX; R = rosiglitazone - no differentiation + minimal differentiation ++ low differentiation +++ moderate differentiation ++++ high differentiation | | |

In another study, we asked whether rosiglitazone could still potentiate fat cell differentiation when added after differentiation had already been partially induced by the combination of insulin, IBMX, and dexamethasone. Thus, human primary preadipocytes were isolated and induced to partially differentiate by the addition of insulin, IBMX, and dexamethasone for the first 8 days. Rosiglitazone was then added to some of the cultures, and incubation was continued until day 15. Images of cultured cells and of their produced lipid droplets were acquired by an inverted microscope between the twelfth and the fifteenth day of the study.

The results of this study are given in table 5 below.

**Table 5**

| Source of cells | Treatment* | Degree of differentiation |
|---|---|---|
| **facial** | **-** | **-** |
| | **I +D+X** | **++** |
| | **I+D+X (+R/8^{th} day)** | **++++** |
| **abdominal** | **-** | **-** |
| | **I +D+X** | **+** |
| | **I+D+X (+R/8^{th} day)** | **+++** |

| | | |
|---|---|---|
| I - insulin; D = dexamethasone; X = IBMX; R = rosiglitazone - no differentiation + minimal differentiation ++ low differentiation +++ moderate differentiation ++++ high differentiation | | |

This example demonstrated that a PPAR-g agonist appears to be required for optimal differentiation of fat cells *in vitro.*

### Example 3: Facial cells retain their ability to differentiate through multiple subpassages to a greater extent than do abdominal preadipocytes

Human primary preadipocytes were isolated and cultured as described above. Facial pre-adipocytes (three different preparations) differentiated to a higher degree than any abdominal preadipocyte preparations. The response to any of the adipogenic molecules was similar in both types of preadipocytes, but the magnitude of the response was more pronounced in the facial preadipocytes. In addition, preadipocytes from human subcutaneous abdominal skin demonstrated a minimal differentiation ability after they were split into new culture plates for the 5th time (5th passage), and their ability to differentiate was substantially impaired. Facial preadipocytes were not substantially impaired in their ability to differentiate even after the 10th passage, and behaved similar to cells from the 3rd passage.

The results of this study are given in table 6 below.

**Table 6**

| Source of cells | Additions to cells^{*} | Degree of differentiation | Passage #^{*} |
|---|---|---|---|
| **facial** | **-** | **-** | **-** |
| | **I+D+X** | **++** | **P3** |
| | **I+D+X+R** | **++++** | **P3** |
| | **I+D+X+R** | **++++** | **P5** |
| | **I+D+X+R** | **++++** | **P10** |
| **Abdominal** | **-** | **-** | **-** |
| | **I+D+X** | **+** | **P3** |
| | **I+D+X+R** | **+++** | **P3** |
| | **I+D+X+R** | **+** | **P5** |

| | | | |
|---|---|---|---|
| I - insulin; D = dexamethasone; X = IBMX; R = rosiglitazone - no differentiation + minimal differentiation ++ low differentiation +++ moderate differentiation ++++ high differentiation | | | |

This example demonstrated that facial and abdominal preadipocytes have different differentiation capacities.

### Example 4: Rosiglitazone is superior to linoleic acid in inducing preadipocyte differentiation

Human primary preadipocytes were isolated and cultured as described in Example 2. All agents (IBMX, dexamethasone, insulin, and Linoleic acid) were added to the media 24 hr after the cells were seeded. The media were changed three days later with fresh preparations of the respective agents. After the seventh day in culture, IBMX and Linoleic acid were removed and the cells were kept for 5 to 10 more days for observation of lipid droplet formation. Between the twelfth and the fifteenth day, images of cultured cells and their produced lipid droplets were acquired by an inverted microscope. Subsequently, the cultures were incubated with oil red O (a dye that binds to neutral lipids); the dye was then extracted and quantified by spectrophotometry. The results given in table 7 below are a summary from two separate experiments:

**Table 7**

| Source of cells | Treatment^{*} | Degree of differentiation |
|---|---|---|
| **facial** | **-** | **-** |
| | **I+L** | **-** |
| | **I+D+X** | **++** |
| | **I+D+X+L** | **+++** |
| **abdominal** | **-** | **-** |
| | **I+L** | **-** |
| | **I+D+X** | **+** |
| | **I+D+X+L** | **++** |

| | | |
|---|---|---|
| I - insulin; D = dexamethasone; X = IBMX; L = linoleic acid was used at 25µM. - no differentiation + minimal differentiation ++ low differentiation +++ moderate differentiation ++++ high differentiation | | |

This example shows that linoleic acid, a physiological ligand of PPAR gamma, can enhance adipogenesis of preadipocytes when added in the media (at 5-50 µM ) in addition to IBMX, insulin and dexamethasone. However, Linoleic acid alone could not affect the differentiation process, without IBMX and dexamethasone (DEX). Therefore, Linoleic acid is not as adipogenic as rosiglitazone, which has the unexpected potential to stimulate differentiation of preadipocytes even in the absence of IBMX and dexamethasone.

### conclusions:

Linoleic acid can also be used to induce differentiation of the subcutaneous fat cells. Rosiglitazone will be more efficacious in areas of facial subcutaneous fat since the preadipocytes in the face are more responsive to this drug. Therefore, very low doses of rosiglitazone can be possibly used in local injections.

### Example 5: Rosiglitazone is superior to natural extracts (Pulpactyl, Einkorn) in inducing preadipocyte differentiation

Pulpactyl is an extract from Artemisia abrotanum (common name: Southernwood), and is claimed to have a PPAR gamma activity. Einkorn, an extract from Triticum monococcum, is known for its ability to enhance lipid production. The ability of these extracts to induce facial preadipocyte differentiation was evaluated.

Human primary preadipocytes were isolated and cultured as described in Example 2. All agents (IBMX, dexamethasone, insulin, and natural extracts) were added to the media 24 hr after the cells were seeded. The media were changed three days later with fresh preparations of the respective agents. After the seventh day in culture, IBMX and natural extracts were removed and the cells were kept for 5 to 10 more days for observation of lipid droplet formation. Between the twelfth and the fifteenth day, images of cultured cells and their produced lipid droplets were acquired by an inverted microscope. Subsequently, the cultures were incubated with oil red O (a dye that binds to neutral lipids); the dye was then extracted and quantified by spectrophotometry. The results given in tables **xxx** and **xxxx** below are from both facial and abdominal preadipocyte experiments:

**Table 8**

| Source of cells | Treatment^{*} | Degree of differentiation |
|---|---|---|
| **facial** | **-** | **-** |
| | **I+L** | **-** |
| | **I+D+X** | **++** |
| | **I+D+X+PP** | **+++** |
| | **I+D+X+R** | **++++** |
| **abdominal** | **-** | **-** |
| | **I+L** | **-** |
| | **I+D+X** | **+** |
| | **I+D+X+PP** | **++** |
| | **I+D+X+R** | **+++** |
| | | |

**Table 9**

| Source of cells | Treatment^{*} | Degree of differentiation |
|---|---|---|
| **facial** | **-** | **-** |
| | **I+L** | **-** |
| | **I+D+X** | **++** |
| | **I+D+X+EinK** | **+++** |
| | **I+D+X+R** | **++++** |
| **abdominal** | **-** | **-** |
| | **I+L** | **-** |
| | **I+D+X** | **+** |
| | **I+D+X+EinK** | **++** |
| | **I+D+X+R** | **+++** |

| | | |
|---|---|---|
| I - insulin; D = dexamethasone; X = IBMX; PP = Pulpactyl extract (0.25%); EinK = Einkorn extract (used at 1%). - no differentiation + minimal differentiation ++ low differentiation +++ moderate differentiation ++++ high differentiation | | |

This example shows that certain natural extracts (at 0.001-20%)can enhance adipogenesis of preadipocytes when combined with IBMX, insulin and dexamethasone. However, the natural extracts did not affect the differentiation process to the same degree as rosiglitazone. Natural extracts such as Pulpactyl and Einkorn can also be used to induce differentiation of subcutaneous fat cells.

### Example 5. Combination of Rosiglitazone and dermal filling material

A study was initiated to examine the effects of a subcutaneous injection of a collagen filler combined with rosiglitazone on the subcutaneous fat. Evolence is a proprietary, cross-linked collagen product, marketed by Colbar, a J&J subsidiary. Samples of Evolence will be incubated in vitro with rosiglitazone under various conditions, which may allow non-covalent interactions between the compound and the collagen. The mixtures will then be injected subcutaneously into the backs of the animals. Alternatively, rosiglitazone might be injected at the same time of Evolence injection. Groups of control rats will be uninjected, injected with saline alone, or injected with either Evolence or rosiglitazone alone. After several weeks, the rats will be sacrificed. Full-thickness biopsies of the injected areas will then be taken, and histologically stained with hematoxylin and eosin, and then examined microscopically for the thickness of the subcutaneous fat layer, the distribution of adipocytes, and the texture and health status of the skins. In addition, the size of individual adipocytes will be measured by image analysis. We expect this study to show an improvement in skin fat parameters, which could be translated into a visible aesthetic benefit, upon rosiglitazone treatment. We expect the combination of dermal filler and rosiglitazone to result in both filler and fat layer effects, with the potential to improve skin defects to best degree.

## Claims

1. A subcutaneous deliverable composition comprising an agonist of the peroxisome proliferator-activated receptor-gamma, and a pharmaceutically acceptable carrier for use in treating the appearance of a facial skin defect in a mammalian subject wherein said use comprises (a) identifying an area of facial skin in need of the treatment in said subject; (b) delivering a safe and cosmetically effective amount of said composition subcutaneously to said area of skin, wherein said delivery results in a reduction of said appearance of facial skin defect.

2. The composition of claim 1, wherein said facial skin defect is selected from the group consisting of scarred skin, wrinkled skin, furrowed skin, folding skin, sagging skin, atrophy from disease or trauma, defects secondary to skin grafting or other surgically-induced irregularities, and irregularity of skin.

3. The composition of claim 1 or claim 2, wherein said agonist of the peroxisome proliferator-activated receptor-gamma is selected from the group consisting of rosiglitazone, ciglitazone troglitazone, englitazone, pioglitazone, linoleic acid, oxidized metabolites of linoleic and arachidonic acid, and the mixtures thereof.

4. The composition of claim 3, wherein said agonist of the peroxisome proliferator-activated receptor-gamma is rosiglitazone.

5. The composition of any preceding claim, wherein said agonist of the PPAR-γ has a concentration range from about 0.00001% to about 5% by weight.

6. The composition of claim 5, wherein said agonist of the PPAR-γ has a concentration range from about 0.001 % to about 0.1% by weight.

7. The composition of claim 6, wherein said agonist of the PPAR-γ has a concentration range from about 0.01% to about 0.1 % by weight.

8. The composition of any preceding claim, wherein said composition further comprises a dermal filling material selected from the group consisting of collagen; cross-linked collagen, hyaluronic acid, poly lactic acid, calcium hydroxyl apatite, cells, minced tissues, autologous transplanted cells or tissues, being intact or fragmented, gelatin, and the mixtures thereof.

9. The composition of claim 8, wherein said cross-linked collagen is cross-linked with one or more sugars.

10. A subcutaneous deliverable composition comprising an agonist of the peroxisome proliferator-activated receptor-gamma, and a pharmaceutically acceptable carrier for use in facial contouring in a mammalian subject, wherein said use comprises:
a) identifying an area of facial skin in need of the treatment in said subject; b) delivering a safe and cosmetically effective amount of said composition subcutaneously to said area of skin, wherein said delivery results in an improvement of facial contour around said area of facial skin.

11. A subcutaneous deliverable composition suitable for treating an appearance of skin defect in a mammalian subject, said deliverable composition comprising: a) an agonist of the peroxisome proliferators-activated receptor-gamma, b) a dermal filling material; and c) a pharmaceutically acceptable carrier.

12. The composition of claim 11, wherein said agonist of the peroxisome proliferator-activated receptor-gamma is rosiglitazone.

13. The composition of claim 11 or claim 12, wherein said dermal filling material is selected from the group consisting of collagen; cross-linked collagen, hyaluronic acid, poly lactic acid, calcium hydroxyl apatite, cells, minced tissues, autologous transplanted cells or tissues, being intact or fragmented, gelatin, and the mixtures thereof.

14. The composition of claim 13, wherein said cross-linked collagen is cross-linked with one or more sugars.
